# EUROPEAN PATENT APPLICATION

(11) **EP 4 371 990 A1**
(43) Date of publication of application: **22.05.2024**
(21) Application number: 22841801.8
(22) Date of filing: 23.05.2022
(51) Int. Cl.: C07D 513/04

(54) **METHOD FOR PRODUCING AROMATIC DIHALOGEN COMPOUND**

(30) Priority: 12.07.2021 JP 2021115028
(71) Applicant: TOYOBO CO., LTD., Osaka-shi, Osaka 530-0001 (JP)
(72) Inventor: SAKAMOTO, Yasuhiro, Otsu-shi, Shiga 520-0292 (JP)
(74) Representative: Müller-Boré & Partner Patentanwälte PartG mbB
(86) International application number: PCT/JP2022/021138
(87) International publication number: WO 2023/286454

(57) **Abstract**

An object of the present invention is to provide a method for industrially producing a high purity aromatic dihalogen compound from an aromatic diamine compound without the use of tert-butyl nitrite, which is a designated substance. A method for producing an aromatic dihalogen compound, comprising a reaction step 1 of reacting an aromatic diamine compound, an alkyl nitrite compound, and a halogenation reagent, wherein the reaction step 1 is conducted at a reaction temperature of 35°C or higher; and the alkyl nitrite compound is at least one selected from the group consisting of ethyl nitrite, hexyl nitrite, and amyl nitrite.

## Description

### TECHNICAL FIELD

The present invention relates to a method for industrially producing an aromatic dihalogen compound. The aromatic dihalogen compound is a useful compound for application in organic electronic materials.

### BACKGROUND ART

In conventional techniques, an aromatic dihalogen compound is synthesized by reacting an aromatic diamine compound with a halogenation reagent, such as iodine or cupric bromide, at room temperature in the presence of tert-butyl nitrite, as described in Patent Documents 1 and 2.
Patent Document 1: JP-B2-4908882
Patent Document 2: WO 2014/031295

### SUMMARY OF THE INVENTION

### PROBLEMS TO BE SOLVED BY THE INVENTION

However, the methods disclosed in Patent Documents 1 and 2 required the use of tert-butyl nitrite. Tert-butyl nitrite is classified as a designated substance and requires stringent handling and management. In addition, tert-butyl nitrite has high toxicity. Therefore, it has been anticipated to develop a reaction capable of producing a high purity aromatic dihalogen compound with a non-designated alkyl nitrite.

The present invention has been done to solve such problems of the conventional techniques. It is an object of the present invention to provide a method for industrially producing a high-purity aromatic dihalogen compound with a non-designated alkyl nitrite compound.

### SOLUTION TO THE PROBLEMS

As a result of earnest studies to solve above problems, the present inventors have arrived at the present invention: i.e., a method for producing an aromatic dihalogen compound with at least one non-designated alkyl nitrite compound selected from the group consisting of ethyl nitrite, hexyl nitrite, and amyl nitrite. These are nitrite ester compounds but are not classified as designated drugs.

[1] A method for producing an aromatic dihalogen compound, comprising a reaction step 1 of reacting an aromatic diamine compound, an alkyl nitrite compound, and a halogenation reagent, wherein the reaction step 1 is conducted at a reaction temperature of 35°C or higher; and, the alkyl nitrite compound is at least one selected from the group consisting of ethyl nitrite, hexyl nitrite, and amyl nitrite.
[2] The method for producing the aromatic dihalogen compound according to [1], wherein the method further comprises a reaction step 2 after the completion of the reaction step 1, and the reaction step 2 is conducted at a higher temperature than the reaction step 1.
[3] The method for producing the aromatic dihalogen compound according to [1] or [2], wherein the reaction step 2 is conducted at a temperature of 60°C or higher.
[4] The method for producing the aromatic dihalogen compound according to any one of [1] to [3], wherein the aromatic diamine compound has a structure represented by Formula (1) and the aromatic dihalogen compound has a structure represented by Formula (2): 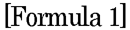 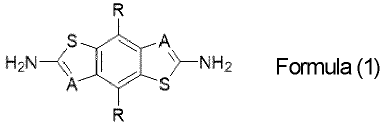 wherein each R independently represents a hydrogen atom, a halogen atom, or an alkyl group and each A independently represents a nitrogen atom or an oxygen atom in Formula (1); 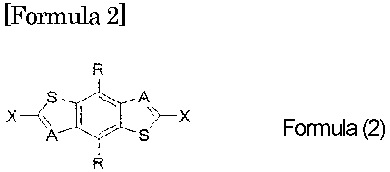 wherein both of R and A represent the same as above and each X independently represents a halogen atom in Formula (2).
[5] The method for producing the aromatic dihalogen compound according to any one of [1] to [4], wherein the halogenation reagent is at least one selected from the group consisting of iodine, bromine, chlorine, a copper halide, and a quaternary ammonium halide salt.

### ADVANTAGEOUS EFFECTS OF THE INVENTION

According to the present invention, a high-purity aromatic dihalogen compound can be produced industrially by reacting an aromatic diamine compound with a non-designated alkyl nitrite compound under appropriate conditions; this eliminates the need for tert-butyl nitrite, which is highly toxic and necessitates stringent handling and management.

### DESCRIPTION OF EMBODIMIENTS

Hereinafter, the present invention will be described in detail.

The method for producing aromatic dihalogen compounds of the present invention comprises a reaction step 1 of reacting an aromatic diamine compound, an alkyl nitrite compound, and a halogenation reagent, wherein the alkyl nitrite compound is at least one selected from the group consisting of ethyl nitrite, hexyl nitrite, and amyl nitrite. Hereinafter, the alkyl nitrite compound may be referred to as a specific alkyl nitrite compound.

The aromatic diamine compound is not particularly limited if the aromatic diamine compound contains an aromatic ring and 2 amino groups. The aromatic diamine compound preferably contains 2 amino groups each directly bonded to an aromatic ring, and more preferably is a compound represented by Formula (1). The aromatic dihalogen compound is not particularly limited if the aromatic dihalogen compound contains an aromatic ring and 2 halogen atoms. The aromatic dihalogen compound preferably contains 2 halogen atoms each directly bonded to an aromatic ring, and is more preferably a compound represented by Formula (2). The aromatic diamine compound and the aromatic dihalogen compound have corresponding structures with one another.

In above Formulae (1) and (2), R, A, and X are defined as follows. In Formula (1), each R independently represents a hydrogen atom, a halogen atom, or an alkyl group. A halogen atom represented by R is preferably an iodine atom, a bromine atom, or a chlorine atom. The alkyl group can be a linear alkyl group or a branched alkyl group. The alkyl group has a carbon number ranging preferably from 1 to 30 and more preferably from 5 to 20. Each A independently represents either a nitrogen atom or an oxygen atom. Both two A are preferably nitrogen atoms or oxygen atoms, and more preferably nitrogen atoms. In Formula (2), R and A represent the same as above, and each X independently represents a halogen atom. A halogen atom represented by X is preferably an iodine atom, a bromine atom, or a chlorine atom.

Ta synthesize the aromatic dihalogen compound, the aromatic diamine compound, the halogenation reagent, and the alkyl nitrite compound are charged in a reaction chamber and reacted.

To synthesize the aromatic dihalogen compound, it is necessary to employ at least one alkyl nitrite compound selected from the group consisting of ethyl nitrite, hexyl nitrite, and amyl nitrite. These specific alkyl nitrite compounds are not classified as designated substances. In accordance with the Act on Securing Quality, Efficacy and Safety of Products Including Pharmaceuticals and Medical Devices, the term "designated substance" is defined as a substance with a high probability of stimulating or suppressing effects on central nervous system or hallucinatory effects (including maintaining or intensifying such effects), and which could cause health and hygiene hazard in the event that such a substance is used in the human body. The designated substances are listed in Order of the Ministry of Health, Labour and Welfare as Referred to in Item (xiiii) of the Act of 2007, which outlines substances designated by Article 2(15) of the Act on Securing Quality, Efficacy and Safety of Products Including Pharmaceuticals and Medical Devices and medical purposes determined by Article 76-4 of the Act. While legal regulations differ from one country to another, tert-butyl nitrite is classified as a designated substance under Japanese law. In any case, the use of tert-butyl nitrite should be avoided due to its toxicity. In contrast, the use of the specific alkyl nitrite compounds does not require complex and special regulations for storage and management as well as special abatement system, and the specific alkyl nitrite compounds can be handled safely.

These specific alkyl nitrite compounds may be used alone or in a combination of 2 or more. The amount of the alkyl nitrite compound is preferably 1.5 mol or more with respect to 1 mol of the aromatic diamine compound. The amount is more preferably 2 mol or more, and further preferably 2.5 mol or more, because the specific alkyl nitrite compound used in such an amount may cause an increase in the reaction rate and thus the reaction time can be shortened. However, the excessive amount of the specific alkyl nitrite compound can lead to saturation of the reaction rate enhancement effect, complicating post-treatment processes; therefore, the amount is preferably 10 mol or less, more preferably 6 mol or less, and further preferably 4 mol or less. In case where 2 or more of the specific alkyl nitrite compounds are used in combination, the total amount of the specific alkyl nitrite compounds used should satisfy the amount of the specific alkyl nitrite compound specified above.

A general halogenation reagent may be employed as the halogenation reagent. Among the general halogenation reagents, iodine, bromine, chlorine, a copper halide, a quaternary ammonium halide salt, and N-halogenated succinimide are preferred, and at least one selected from the group consisting of iodine, bromine, chlorine, a copper halide, and a quaternary ammonium halide salt is particularly preferred The copper halide is not particularly limited. Examples of the copper halide include cuprous chloride, cupric chloride, cuprous bromide, cupric bromide, and cuprous iodide, and cupric bromide is preferred among them. Examples of the quaternary ammonium halide salt include tetra-n-butylammonium bromide (TBAB), tetrabutylammonium chloride (TBAC), and tetrabutylammonium iodide (TBAI). Examples of the N-halogenated succinimide include N-bromosuccinimide (NBS), N-chlorosuccinimide (NCS), and N-iodosuccinimide (NIS). These halogenation reagents may be used alone or in a combination of 2 or more. The amount of the halogenation reagent is preferably from 1.5 mol to 6 mol and more preferably from 2 mol to 4 mol with respect to 1 mol of the aromatic diamine compound.

In order to accelerate halogenation, an organic sulfonic acid compound may also be employed The organic sulfonic acid compound is not particularly limited, and examples of the organic sulfonic acid compound include p-toluenesulfonic acid, benzenesulfonic acid, methanesulfonic acid, and trifluoromethanesulfonic acid In case where the organic sulfonic acid compound is employed, the amount of the organic sulfonic acid compound is preferably from 1.5 mol to 6 mol, and more preferably from 2 mol to 4 mol with respect to 1 mol of the aromatic diamine compound.

A solvent (reaction solvent) may be used in the reaction. Importantly, the solvent should not impede the reaction. Examples of preferred solvent include acetonitrile (AN), N,N-dimethylformamide (DMF), dimethyl sulfoxide (DMSO), N,N-dimethylacetamide (DMAC), N-methyl-2-pyrrolidone (NMP), chloroform, ethyl acetate, hexane, heptane, toluene, and tetrahydrofuran. These solvents may be used alone or in a combination of 2 or more. The amount of the solvent is preferably from 2 to 100 parts by mass, more preferably from 4 to 50 parts by mass, and further preferably from 6 to 30 parts by mass with respect to 1 part by mass of the aromatic diamine compound.

Compared to tert-butyl nitrite, the specific alkyl nitrite compounds exhibit lower reactivity. Therefore, the method for producing the aromatic dihalogen compound from the aromatic diamine compound, the alkyl nitrite compound, and the halogenation reagent should comprise the reaction step 1, which is conducted at least at a reaction temperature of 35°C or higher. The reaction temperature in the reaction step 1 is preferably 40°C or higher, and more preferably 45°C or higher. The reaction temperature in the reaction step 1 is preferably 70°C or lower, more preferably 60°C or lower, and further preferably lower than 60°C. The reaction time in the reaction step 1 is preferably 30 minutes or more, more preferably 1 hour or more, and further preferably 2 hours or more. The reaction time in the reaction step 1 is preferably 5 hours or less, more preferably 4 hours or less, and further preferably 3 hours or less. The reaction step 1 under these conditions enables the production of high-quality aromatic dihalogen compound.

The method according to the present invention further comprises a reaction step 2 after the completion of the reaction step 1, and the reaction step 2 is conducted at a higher temperature than the reaction step 1. By conducting the reaction step 2, the quality of the aromatic dihalogen compound can be further improved. The reaction temperature in the reaction step 2 is not particularly limited as long as the reaction step 2 is conducted at a higher temperature than the reaction step 1. The reaction temperature in the reaction step 2 is preferably 60°C or higher, more preferably 70°C or higher, and further preferably 80°C or higher. The reaction temperature in the reaction step 2 is preferably 130°C or lower, more preferably 120°C or lower, and further preferably 110°C or lower. The reaction time in the reaction step 2 is preferably 1 hour or more, more preferably 3 hours or more, and further preferably 5 hours or more. The reaction time in the reaction step 2 is preferably 20 hours or less, more preferably 18 hours or less, and further preferably 16 hours or less. By further conducting the reaction step 2 under these conditions after completing reaction step 1, the production of impurities (byproducts) can be reduced, resulting in further improvement in the quality of the aromatic dihalogen compound.

A difference in the reaction temperatures between the reaction step 1 and the reaction step 2 is preferably 25°C or higher, more preferably 30°C or higher, and further preferably 40°C or higher. A difference in the reaction temperatures is preferably 90°C or lower, more preferably 80°C or lower, and further preferably 70°C or lower. By providing the difference in the reaction temperatures, the generation of impurities can be reduced, and the quality of the aromatic dihalogen compound can be further improved.

Once the reaction is finished, the resulting aromatic dihalogen compound undergoes several post-reaction processes, including filtration to separate solid from liquid. This process of separating solid from liquid allows for the easy extraction of a crude product. If necessary, the crude product is then preferably subjected to further purification steps like crystallization, suspension washing, and recrystallization. These purification procedures are highly effective in producing a high-purity aromatic dihalogen compound.

The suspension washing can be conducted with the same solvent that is used as the reaction solvent. For recrystallization, solvents such as DMSO, NMP, DMF, and DMAC are suitable due to their solubility properties.

### EXAMPLES

The present invention will be described more specifically by the following Examples; however, the scope of the present invention is not limited to the Examples.

### <Measurement of purity by high performance liquid chromatography (HPLC) >

Purity of the aromatic dihalogen compound was measured by HPLC under the following measurement conditions. The measurement results were presented as percentage areas.
Measurement system: high performance liquid chromatograph (Prominence LC20 system, manufactured by Shimadzu Corporation)
Column: Inertsill ODS-3, 5 pm, 4.6 × 250 mm (manufactured by GL Sciences Inc.). A column oven was set to 40°C.
Measurement wavelength: UV detector, 290 nm
Flow rate: 1.0 ml/min
Mobile phase composition: A=acetonitrile, B=0.01M aqueous solution of K₂HPO₄ (pH=4)
Gradient program
   From 0 to 5 minutes: isocratic elution with a mobile phase ofA/B=40/60
   From 5 to 7.5 minutes: gradient elution with a mobile phase ofA/B=75/25
   From 7.5 to 35 minutes: isocratic elution with a mobile phase ofA/B=75/25 Sample for measurement: 1 mg of aromatic dihalogen compound was dissolved in 20 ml of DMSO to be measured.
Injection volume: 20 µl
Quality of the aromatic dihalogen compound was evaluated as follows.
   Grade A: HPLC purity was 95% or more.
   Grade B: HPLC purity was 90% or more and less than 95%.
   Grade C: HPLC purity was 85% or more and less than 90%.
   Grade D: HPLC purity was less than 85%.

### Example 1: production of 2,6-dibromobenzo[1,2-d:4,5-d']bisthiazole

In a 200 ml flask, N,N-dimethylformamide (74.0 g) and cupric bromide (27.3 g, 122.4 mmol) were charged and stirred at 20°C. Subsequently, hexyl nitrite (14.2 g, 102.5 mmol) was added. Following 15 minutes of stirring, 2,6-diaminobenzo[1,2-d:4,5-d']bisthiazole (8.0 g, 36.0 mmol) was added gradually over 30 minutes, and the reactants were allowed to react at 40°C for 3 hours. Upon completion of the reaction, an 8.7% by mass of an aqueous solution of hydrogen bromide (48.0 g) was added to the mixture, and crude crystals of 2,6-dibromobenzo[1,2-d:4,5-d']bisthiazole were obtained by filtration. These crude crystals were first rinsed with a 35% by mass of hydrochloric acid, then suspended in dimethyl sulfoxide (80.0 g) for an additional wash. A subsequent wash was conducted by suspending the washed crude crystals in ethyl acetate (40.0 g), followed by vacuum drying at 40°C to give light brown 2,6-dibromobenzo[1,2-d:4,5-d']bisthiazole crystals. The results are shown in Table 1.

### Example 2: 2,6-dibromobenzo[1,2-d:4,5-d']bisthiazole

The production of 2,6-dibromobenzo[1,2-d:4,5-d']bisthiazole was conducted following the same procedure as described in Example 1 under the conditions shown in Table 1. The results are shown in Table 1.

### Example 3: 2,6-dibromobenzo[1,2-d:4,5-d']bisthiazole

The production of 2,6-dibromobenzo[1,2-d:4,5-d']bisthiazole was conducted following the same procedure as described in Example 1 under the conditions shown in Table 1. The results are shown in Table 1.

### Example 4: 2,6-diiodidebenzo[1,2-d:4,5-d']bisthiazole

The production of 2,6-diiodidebenzo[1,2-d:4,5-d']bisthiazole was conducted following the same procedure as described in Example 1 with a reagent shown in Table 1. The results are shown in Table 1.

### Example 5: 2,6-dibromobenzo[1,2-d:4,5-d']bisthiazole

In a 200 ml flask, acetonitrile (63.0 g), p-toluenesulfonic acid monohydrate (16.4 g, 86.3 mmol), tetrabutylammonium bromide (46.4 g, 143.9 mmol), 2,6-diaminobenzo[1,2-d:4,5-d']bisthiazole (8.0 g, 36.0 mmol), and cupric bromide (0.24 g, 1.0 mmol) were charged and stirred at 20°C for 15 minutes. Subsequently, hexyl nitrite (12.5 g, 95.4 mmol) was added gradually over 30 minutes, and the reactants were allowed to react at 40°C for 3 hours. Upon completion of the reaction, crude crystals of 2,6-dibromobenzo[1,2-d:4,5-d']bisthiazole were obtained by filtration. These crude crystals were suspended in 50% by mass of an aqueous solution of acetonitrile (33.6 g) for wash, followed by vacuum drying at 40°C to give light brown 2,6-dibromobenzo[1,2-d:4,5-d']bisthiazole crystals. The results are shown in Table 1.

### Example 6: 2,6-dibromobenzo[1,2-d:4,5-d']bisthiazole

In a 200 ml flask, N,N-dimethylformamide (74.0 g) and cupric bromide (27.3 g, 122.4 mmol) were charged and stirred at 20°C. Subsequently, hexyl nitrite (14.2 g, 102.5 mmol) was added. Following 15 minutes of stirring, 2,6-diaminobenzo[1,2-d:4,5-d']bisthiazole (8.0 g, 36.0 mmol) was added gradually over 30 minutes, and the reactants were allowed to react at 40°C for 3 hours. The mixture was further stirred at 100°C for another 15 hours. Upon completion of the reaction, an 8.7% by mass of an aqueous solution of hydrogen bromide (48.0 g) was added to the mixture, and crude crystals of 2,6-dibromobenzo[1,2-d:4,5-d']bisthiazole were obtained by filtration. These crude crystals were first rinsed with a 35% by mass of hydrochloric acid, then suspended in dimethyl sulfoxide (80.0 g) for an additional wash. A subsequent wash was conducted by suspending the washed crude crystals in ethyl acetate (40.0 g), followed by vacuum drying at 40°C to give light brown 2,6-dibromobenzo[1,2-d:4,5-d']bisthiazole crystals. The results are shown in Table 1.

### Example 7: 2,6-dibromobenzo[1,2-d:4,5-d']bisthiazole

The production of 2,6-dibromobenzo[1,2-d:4,5-d']bisthiazole was conducted following the same procedure as described in Example 1 under the conditions shown in Table 1. The results are shown in Table 1.

### Comparative Example 1: 2,6-dibromobenzo[1,2-d:4,5-d']bisthiazole

The production of 2,6-dibromobenzo[1,2-d:4,5-d']bisthiazole was conducted following the same procedure as described in Example 1 under the conditions shown in Table 1. The results are shown in Table 1.

### Comparative Example 2: 2,6-dibromobenzo[1,2-d:4,5-d']bisthiazole

The production of 2,6-dibromobenzo[1,2-d:4,5-d']bisthiazole was conducted following the same procedure as described in Example 1 under the conditions shown in Table 1. The results are presented in Table 1.

### Comparative Example 3: 2,6-dibromobenzo[1,2-d:4,5-d']bisthiazole

The production of 2,6-dibromobenzo[1,2-d:4,5-d']bisthiazole was conducted following the same procedure as described in Example 1 under the conditions shown in Table 1. The results are presented in Table 1.

In Examples 1 to 7, high purity aromatic dihalogen compounds were produced safely with non-designated alkyl nitrite compounds under appropriate reaction conditions. In Comparative Example 1, the reaction temperature was low and the resulting aromatic dihalogen compound had lower purity. In Comparative Example 2, tert-butyl nitrite was used and the resulting aromatic dihalogen compound had decreased quality. In addition, tert-butyl nitrite is a designated substance and the use of it causes safety hazards. Furthermore, a large-scale industrial use of tert-butyl nitrite requires a stringent management and abatement system, making handling of it complicated.

**[Table 1]**

| | | | Example 1 | Example 2 | Example 3 | Example 4 | Example 5 | Example 6 | Example 7 | Comparative Example 1 | Comparative Example 2 | Comparative Example 3 |
|---|---|---|---|---|---|---|---|---|---|---|---|---|
| Aromatic diamine compound represented by Formula (1) | | X in Formula (1) | Br | Br | Br | I | Br | Br | Br | Br | Br | Br |
| | | 4 in Formula (1) | N | N | N | N | N | N | N | N | N | N |
| | | R in Formula (1) | H | H | H | H | H | H | H | H | H | H |
| | | (g) | 8.0 | 8.0 | 8.0 | 8.0 | 8.0 | 8.0 | 8.0 | 8.0 | 8.0 | 8.0 |
| | | (mol) | 36.0 | 36.0 | 36.0 | 36.0 | 36.0 | 36.0 | 36.0 | 36.0 | 36.0 | 36.0 |
| Solvent | | | DMF | DMF | DMF | AN | AN | DMF | AN | DMF | DMF | AN |
| | | (g) | 74.0 | 74.0 | 74.0 | 189.6 | 63.0 | 74.0 | 74.0 | 74.0 | 74.0 | 63.0 |
| | | Mass ratio (with respect to raw material) | 9.3 | 9.3 | 9.3 | 23.7 | 7.9 | 9.3 | 9.3 | 9.3 | 9.3 | 7.9 |
| Halogenation reagent | Halogenation reagent 1 | | Cupric bromide | Cupric bromide | Cupric bromide | Iodine | Cupric bromide | Cupric bromide | Cupric bromide | Cupric bromide | Cupric bromide | Cupric bromide |
| | | (mol) | 122.4 | 122.4 | 122.4 | 122.4 | 1.0 | 122.4 | 122.4 | 122.4 | 122.4 | 122.4 |
| | | Mole ratio (with respect to raw material) | 3.4 | 3.4 | 3.4 | 3.4 | 0.03 | 3.4 | 3.4 | 3.4 | 3.4 | 3.4 |
| | Halogenation reagent 2 | | - | - | - | - | TBAB | - | - | - | - | - |
| | | (mol) | - | - | - | - | 143.9 | - | - | - | - | - |
| | | Mole ratio (with respect to raw material) | - | - | - | - | 400 | - | - | - | - | - |
| | Total mole ratio (with respect to raw material) | | 3.4 | 3.4 | 3.4 | 3.4 | 4.0 | 3.4 | 3.4 | 3.4 | 3.4 | 3.4 |
| Acid catalyst | | | | | | | p- Toluenesulfonic acid | | | | | |
| | | (mol) | | | | | 86.3 | | | | | |
| | | Moie ratio (with respect to raw material) | | | | | 2.40 | | | | | |
| Alkyl nitrite compound | | | Hexyl nitrite | 15% Ethyl nitrite | ArrM nitrite | ArrM nitrite | Hexyl nitrite | Hexyl nitrite | Hexyl nitrite | Hexyl nitrite | Tert-butyl nitrite | Tert-butyl nitrite |
| | | (mol) | 102.5 | 102.5 | 102.5 | 102.5 | 95.4 | 102.5 | 102.5 | 102.5 | 102.5 | 102.5 |
| | | Mole ratio (with respect to raw material) | 2.8 | 2.8 | 2.8 | 2.8 | 2.7 | 2.8 | 2.8 | 2.8 | 2.8 | 2.8 |
| Reaction step 1 | | Temperature (°C) | 40 | 40 | 40 | 40 | 40 | 40 | 40 | 20 | 40 | 40 |
| | | Time (h) | 3 | 3 | 3 | 1 | 3 | 3 | 3 | 3 | 3 | 3 |
| Reaction step 2 | | Temperature (°C) | - | - | - | - | - | 100 | - | - | - | - |
| | | Time (h) | - | - | - | - | - | 15 | - | - | - | - |
| Aromatic dihalogen compound represented by Formula (2) | | Appearance | Light brown crystals | Light brown crystals | Light brown crystals | Red crystals | Light brown crystals | Light brown crystals | Light brown crystals | Light brown crystals | Light brown crystals | Light brown crystals |
| | | Yield (g) | 10.8 | 10.3 | 10.9 | 107 | 10.3 | 10.7 | 10.9 | 10.5 | 11.0 | 11.9 |
| | | Yield as percentage (%) | 85.6 | 81.5 | 86.1 | 75.0 | 82.1 | 84.9 | 86.3 | 83.0 | 87.2 | 94.6 |
| | | HPLC purity(%) | 93.8 | 93.1 | 93.5 | 90.2 | 95.9 | 97.7 | 88.3 | 76.6 | 86.3 | 94.4 |
| | | Quality | Grade B | Grade B | Grade B | Grade B | Grade A | Grade A | Grade C | Grade D | Grade C | Grade B |
| Safety | | | Good | Good | Good | Good | Good | Good | Good | Good | Bad | Bad |

### INDUSTRIAL APPLICABILITY

According to the method described herein, a high purity aromatic dihalogen compound can be produced safely with a non-designated alkyl nitrite compound. The aromatic dihalogen compound obtained through this method of the present invention serves as an important substrate for the Suzuki-Miyaura coupling reaction and the Heck reaction. The resulting aromatic dihalogen compounds are useful for their usefulness in organic electronic materials.

## Claims

1. A method for producing an aromatic dihalogen compound, comprising a reaction step 1 of reacting an aromatic diamine compound, an alkyl nitrite compound, and a halogenation reagent,
wherein the reaction step 1 is conducted at a reaction temperature of 35°C or higher; and
the alkyl nitrite compound is at least one selected from the group consisting of ethyl nitrite, hexyl nitrite, and amyl nitrite.

2. The method for producing the aromatic dihalogen compound according to claim 1, wherein the method further comprises a reaction step 2 after the completion of the reaction step 1, and the reaction step 2 is conducted at a higher temperature than the reaction step 1.

3. The method for producing the aromatic dihalogen compound according to claim 1 or 2, wherein the reaction step 2 is conducted at a temperature of 60°C or higher.

4. The method for producing the aromatic dihalogen compound according to claim 1 or 2, wherein the aromatic diamine compound has a structure represented by Formula (1) and the aromatic dihalogen compound has a structure represented by Formula (2): 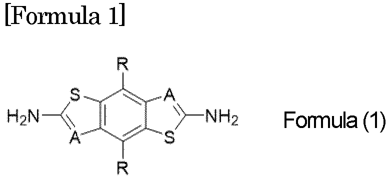 wherein each R independently represents a hydrogen atom, a halogen atom, or an alkyl group and each A independently represents a nitrogen atom or an oxygen atom in Formula (1); 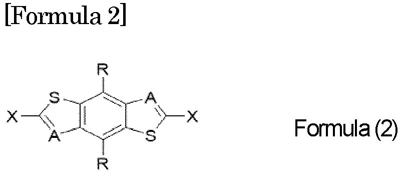 wherein both of R and A represent the same as above and each X independently represents a halogen atom in Formula (2).

5. The method for producing the aromatic dihalogen compound according to claim 1 or 2, wherein the halogenation reagent is at least one selected from the group consisting of iodine, bromine, chlorine, a copper halide, and a quaternary ammonium halide salt.
